(19) Europäisches Patentamt
European Patent Office
Office européen des brevets

(11) Numéro de publication: **0 060 754**
**B1**

(12) # FASCICULE DE BREVET EUROPEEN

(45) Date de publication du fascicule du brevet: **19.06.85**

(51) Int. Cl.$^4$: **C 07 C 149/06**

(21) Numéro de dépôt: **82400368.5**

(22) Date de dépôt: **03.03.82**

(54) **Perfectionnement à la synthèse photoinitiée de mercaptans.**

(30) Priorité: **12.03.81 FR 8104956**
**22.02.82 FR 8202849**

(43) Date de publication de la demande:
**22.09.82 Bulletin 82/38**

(45) Mention de la délivrance du brevet:
**19.06.85 Bulletin 85/25**

(84) Etats contractants désignés:
**DE GB NL**

(56) Documents cités:
**FR-A-2 424 907**

**CHEMICAL ABSTRACTS, vol.93, no.2, 14 juillet 1980, page 81, résumé no. 9600y, Columbus, Ohio (US), & Shikisai Kyohaishi 1980, 53(3), 140-5, H. KANEHIRO et al.:"Curable resin comprising an unsaturated cycloacetal compound and a polythiol compound"**

(73) Titulaire: **SOCIETE NATIONALE ELF AQUITAINE (PRODUCTION)**
**Tour Aquitaine**
**F-92400 Courbevoie (FR)**

(72) Inventeur: **Arretz, Emmanuel**
**2 rue de Cagnes**
**F-64000 Pau (FR)**
Inventeur: **Landoussy, Claude**
**12 rue du Pourtalet**
**F-64000 Pau (FR)**
Inventeur: **Mirassou, Alfred**
**F-64230 Poey-de-Lescar (FR)**
Inventeur: **Ollivier, Jean**
**Croix de Buzy**
**F-64260 Arudy (FR)**

(74) Mandataire: **Kohn, Armand**
**5 Avenue Foch**
**F-92380 Garches (FR)**

**EP 0 060 754 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne un procédé perfectionné de production de mercaptans à partir de composés oléfiniques, par la réaction de ces derniers avec de l'hydrogène sulfuré. Elle se rapporte plus spécialement à un nouveau groupe d'initiateurs permettant de réaliser cette réaction sous l'effet de radiations ultraviolettes.

Etant donné l'utilité industrielle bien connue de différents mercaptans, les méthodes de préparation de ces corps ont donné lieu à de nombreux travaux. Un procédé, qui a conduit à des résultats industriels intéressants, réside dans l'action directe de l'hydrogène sulfuré sur des substances éthyléniques, sous l'effet de radiations ultraviolettes. Dans la pratique de ce procédé, on a été conduit à introduire, dans le milieu réactionnel, des agents promoteurs, et en particulier des phosphites organiques, comme décrit, par exemple, dans le brevet U.S. n° 3 050 452. La création photochimique des radicaux SH, devant induire l'addition de l'$H_2S$ sur la double liaison du composé éthylénique, implique l'utilisation des radiations ultraviolettes de courtes longueurs d'onde, ce qui présente certains inconvénients. En effet, on est obligé d'utiliser des réactifs et des solvants particulièrement purs et transparents; d'autre part, le parcours optique dans le réacteur est faible à cause du coefficient d'extinction moléculaire assez fort de l'hydrogène sulfuré; en outre, le mercaptan synthétisé absorbe lui-même des radiations, et cela provoque un ralentissement considérable des réactions pour des degrés d'avancement peu élevés, avec formation de sulfure par addition du mercaptan lui-même sur la double liaison du composé éthylénique. Une amélioration de ce procédé a été apportée par quelques auteurs qui ont trouvé des photo-initiateurs ou des photo-sensibilisants, susceptibles d'être excités dans un domaine de longueurs d'ondes plus grandes. C'est ainsi que le procédé, décrit dans le brevet U.S. n° 4 052 283, utilise des photosensibilisants organiques contenant un ou deux substituants carbonyle. D'autre part, le brevet U.S. n° 4 140 604 décrit des activateurs de la réaction, constitués par des dérivés de l'acétophénone.

Un progrès encore plus important a été accompli par le procédé object de brevet français publié sous le n° 2 424 907: ce procédé conduit à des rendements meilleurs et permet également d'utiliser une bande large de longueur d'onde. Il consiste à ajouter au milieu réactionnel habituel, en tant que promoteur, un dérivé organique d'un élément du groupe V A de la Classification Périodique des Eléments, en même temps que des initiateurs constitués par des benzophénones ou/et des thio-benzophénones, ou bien par un ou plusieurs dérivés de ces aryl-cétones. De telles cétones peuvent d'ailleurs donner, comme promoteurs photo-chimique employés seuls, des résultats meilleurs que d'autres adjuvants connus. Aisi permettent-elles de produire davantage de mercaptan qu'il n'est possible d'obtenir avec, par exemple, des phosphites trialkyliques ou triaryliques seuls, tels que décrits dans le brevet U.S. n° 3 050 452; en outre, alors que selon ce brevet on est obligé d'utiliser de l'ultraviolet de longueurs d'onde plus courtes que 300 nm, il est possible d'opérer avec plus de 300 nm. dans le cas des benzo- ou thiobenzophénones. Les résultats sont remarquablement améliorés lorsque ces benzophénones ou/et thiobenzo-phénones sont employés conjointement avec des phosphites ou avec d'autres dérivés organiques des éléments du groupe V.A.

La présente invention apporte un perfectionnement notable par rapport à tous ces procédés de l'art antérieur, en ce qu'elle permet d'augmenter sensiblement le taux de production du mercaptan à partir d'un composé oléfinique. Elle présente l'avantage de pouvoir être réalisée soit sans solvant soit au sein d'un solvant tel qu'un sulfure organique, un hydrocarbure ou un éther. Dans le cas d'un sulfure organique, l'avantage est d'autant plus marqué que ce sulfure peut être celui qui est obtenu en tant que sous-produit de la réaction elle-même.

Le nouveau procédé suivant l'invention est caractérisé en ce que le milieu réactionnel est additionné d'un dérivé de type xanthénique qui agit comme initiateur de la formation de mercaptan à partir d'un composé non saturé et de $H_2S$, sous l'effet de radiations ultraviolettes.

Les initiateurs suivant l'invention répondent à la formule

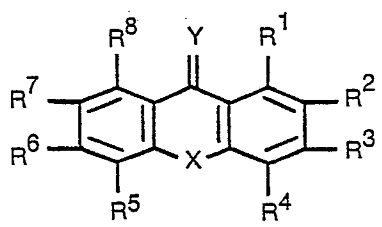

(1)

où Y est O ou S, tandis que X désigne un élément O, S ou Se, un groupe $—CH_2$, $—CH_2CH_2—$, $SO_2$, CO, CS, NR', R' étant un radical hydrocarboné, ou bien un groupement au phosphore tri- ou pentavalent, notamment

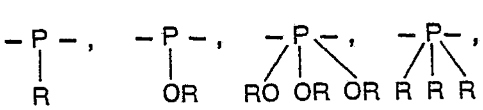

, , , ,

où R désigne un alkyle, un aryle, un H ou un halogène. Les symboles $R^1$ à $R^8$ désignent des atomes ou groupes semblables ou différents, tels que H, halogène, alcoxy, alkyle ou/et aryle.

Le domaine de longueurs d'ondes préféré est de 300 à 400 nm.

Le taux de transformation du composé oléfinique en mercaptan est encore plus élevé si le dérivé xanthénique est ajouté conjointement avec un composé organique d'un élément du group V A de la Classification Périodique, connu en soi comme initiateur de la réaction photochimique en question. Des proportions faible d'initiateurs suffisent, ce qui prouve que le dérivé xanthénique exerce, avec le composé organique du groupe V A, un action synergique.

Bien que des dérivés organiques du phosphore, notamment des phosphites et des phosphines d'alkyles ou d'aryles, soient le plus couramment employés, grâce à leur disponibilité commerciale, l'adjuvant du dérivé xanthénique, suivant l'invention, peut être également un composé organique correspondant du bismuth, de l'arsenic ou de l'antimoine; par exemple, des arsénites d'alkyles ou d'aryles, ou bien des arsines, conviennent en tant qu'adjuvants.

Ces dérivés du type xanthénique permettent d'amplifier l'effet initiateur des agents connus, notamment des phosphites mentionnés plus haut, et de limiter l'action inhibitrice que les sulfures, éventuellement formés par une réaction secondaire, produisent sur la formation du mercaptan recherché. Grâce à cette dernière propriété, l'invention rend possible la fabrication simultanée de mercaptans et de sulfures, ce qui peut être très utile dans certains cas.

Les proportions de dérivé xanthénique, suivant l'invention, à ajouter au milieu réactionnel dépendent de différents facteurs, notamment de la nature des réactifs traités, et de leur concentration dans le milieu, de la présence ou de l'absence du solvant, etc. Le plus souvent, ces proportions sont de l'ordre de 0,0001 à 0,1 mole par litre de milieu réactionnel, et surtout de 0,0003 à 0,001. La proportion de composé de l'élément du group V A est du même ordre.

En ce qui concerne les conditions opératoires, en particulier les températures et les proportions $H_2S$/oléfines, elles sont les mêmes que dans la technique correspondante, déjà connue par des publications antérieures. Il n'y a donc pas lieu de les décrire en détail ici; on notera seulement que les rapports molaires préférés $H_2S$/oléfines sont d'environ 2 à 10, la température étant maintenue entre $-10$ et $+20$°C, des températures d'échelonnant de $-10$° à $-5$° et de $+20$° à $+35$°C étant néanmoins utilisables, bien que moins avantageuses.

La réaction peut être réalisée à pression atmosphérique, mais il est préférable, pour accroître la conversion en mercaptan, d'opérer à des pressions manométriques de 3 à 30 bars.

En ce qui concerne la nature des oléfines dont la transformation en mercaptan peut être améliorée par le procédé de l'invention, on peut citer, à titre d'exemples non limitatifs: éthylène, propylène, butène-1, butène-2, isobutène, pentène-1, pentène-2, hexène-1, heptène-1, octène-1, décène-1, undécène-1, dodécène-1, tétradécène-1, hexadécène-1, octadécène-1, eicosène-1, isopentène-1, méthyl-4-pentène-1, diméthyl-3,6-heptène-1, méthyl-4-butyl-5-décène-4, diphenyl-1,4-butène-2, cyclohéxyl-3-eicosène-6, méthyl-4-pentène-2, triméthyl-2,4,4-pentène-2-cyclopentène, diéthyl-2,5-cyclopentène-cyclohéxène, éthyl-3-cyclohexène, cyclopentène, cyclooctène, vinyl-4-cyclohéxène, esters alkyliques des acides acrylique, méthacrylique, crotonique, allylacétique (penténoïque), octylénique, undécylénique, dodécylénique, octadécylénique, etc.; les esters en question peuvent notamment contenir des alkyles en $C_1$ à $C_{30}$ et plus particulièrement en $C_1$ à $C_8$. Les composés oléfiniques peuvent renfermer plusieurs doubles liaisons, comme c'est par exemple le cas du batadiène ou de l'hexadiène-2,4. Le procédé s'applique également à des composés insaturés porteurs de substituants tels que, par exemple, halogènes, hydroxyles, alcoxy, carboxyles, etc.; il peut ainsi être pratiqué avec des produits comme chlorure d'allyle, alcool allylique, maléates ou fumarates d'alkyles ou d'aryles et composés similaires.

Des composés insaturés à triple liaison sont aussi justiciables du procédé de l'invention: c'est en particulier le cas de alcynes tels qu'acétylène qui conduit au vinyl-mercaptan.

Parmi les composés selon l'invention, particulièrement utiles, on peut citer, par exemple, les composés suivant formule (1) dans laquelle les sites X et Y sont les suivants:

| | X | Y |
|---|---|---|
| xanthone | O | O |
| xanthione | O | S |
| thioxanthone | S | O |
| anthrone | $CH_2$ | O |
| thioanthrone | $CH_2$ | S |
| dibenzosubérone | $CH_2CH_2$ | O |
| thio-dibenzosubérone | $CH_2CH_2$ | S |
| anthraquinone | CO | O |

et leurs dérivés substitués sur les noyaux benzéniques, notamment alkylés, alkoxylés ou halogénés.

Comme on le voit, le cycle médian peut être à 6 ou 7 éléments, cette dernière configuration correspondant à la présence de $—CH_2CH_2—$ en X; si Y est un oxygène, le composé, qui est dans ce cas la dibenzosubérone, se présente à l'état liquide, aisément manipulable, d'ailleurs facilement accessible industriellement. Il en est de même de la thiodibenzo-subérone, dans laquelle Y est un atome de soufre, X étant $—CH_2CH_2—$.

Un avantage particulier des initiateurs selon l'invention réside en ce qu'ils peuvent éventuellement servir sous la pression atmosphérique; bien que, dans ces conditions, le taux de conversion de l'oléfine et la production horaire de mercaptan soient moindres que sous une pression de plusieurs bars, ils sont nettement plus forts qu'avec les initiateurs anciens, tels que phosphites organiques seuls ou des acétophénones employés dans les mêmes conditions. L'absence de produits secondaires, autres que le sulfure correspondant, permet de séparer aisément le mercaptan formé et de recycler l'oléfine et l'hydrogène sulfuré.

L'invention est illustrée par les exemples non limitatifs, qui suivent.

Exemples 1 à 5

La réaction est effectué dans un réacteur cylindrique en acier inoxydable de 245 ml, équipé d'un tube coaxial en quartz contenant une lampe UV, dont le maximum d'émission correspond à la longueur d'onde de 350 nm. La réfrigeration et l'agitation du milieu réactionnel sont assurées par une boucle extérieure, constituée d'une pompe de recirculation et d'un échangeur permettant de maintenir la température du milieu réactionnel à environ 10°C. On effectue une série d'essais dont les paramètres suivants sont maintenus fixes: débit d'hydrogène sulfuré de 240 l/h, débit de dodécène-1 de 336 g/h et pression de 12 bars. Les conditions particulières à chaque essai et les résultats obtenus sont présentés dans le tableau suivant.

TABLEAU I

| Exemple n° | Initiateurs et leurs débits en mole/h | Conversion de Dodécène (%) | Production en dodécylmercaptan (g/h) |
|---|---|---|---|
| 1 | α-éthoxy-α-phényl* acétophénone ($2.10^{-3}$) | 65 | 222 |
| 2 | α-éthoxy-α-phényl* acétophénone ($6.10^{-3}$) | 74 | 247 |
| 3 | Benzophénone ($5.10^{-3}$) + tributylphosphite ($5.10^{-3}$) | 77 | 255 |
| 4 | Xanthone ( $10^{-4}$) + tributylphosphite ($5.10^{-3}$) | 80 | 260 |
| 5 | Thioxanthone ($2.10^{-4}$) + tributylphosphite ($5.10^{-4}$) | 85 | 270 |

*Initiateur selon le brevet U.S. n° 4 140 604.

Exemples 6 et 7

Dans le réacteur décrit dans les exemples 1 à 5, on introduit en continu, sous une pression de 8 à 10 bars, sous forme gazeuse, 120 l/h d'hydrogène sulfuré et 60 l/h de propylène et, sous forme liquide, 100 ml d'éther diméthylique du diéthylène glycol. La concentration en photo-initiateurs répond à l'optimum de l'intensité absorbée dans le réacteur. On maintient la température du milieu réactionnel à environ 0°C.

Les résultats des différents essais effectués dans ces conditions sont présentés dans le tableau suivant.

TABLEAU II

| Exemple n° | Initiateur et sa concentration en moles/l | Conversion de Propylène (%) | Production en g/h | |
|---|---|---|---|---|
| | | | n-propyl mercaptan | sulfure de dipropyle |
| 6 | Benzophénone 0,03 | 42,2 | 41,1 | 8 |
| 7 | Thioxanthone 0,03 | 63,6 | 58,1 | 19,9 |

Exemples 8 à 11

Dans les mêmes conditions opératoires que dans les exemples 6 et 7, on utilise la benzophénone ou la thioxanthone, en association avec un phosphite, dont le débit est de $19 \times 10^{-4}$, celui de la benzophénone étant de $33 \times 10^{-4}$ et celui de la thioxanthone de $1 \times 10^{-4}$ mole/heure.

TABLEAU III

| Exemple n° | Initiateurs | Conversion de Propylène (%) | Production en g/h | |
|---|---|---|---|---|
| | | | n-propyl-mercaptan | sulfure de dipropyle |
| 8 | Benzophénone + phosphite de tributyle | 70,9 | 64 | 25 |
| 9 | Thioxanthone + phosphite de tributyle | 81,4 | 72,8 | 34,8 |
| 10 | Thioxanthone + tributylphosphine | 95,5 | 85,1 | 51,2 |
| 11 | Thioxanthone + thiophosphite de tributyle | 69,9 | 67,1 | 27 |

Exemple 12

On opère dans l'appareillage des exemples précédents à une température d'environ −3°C, sous une pression de 9 à 10 bars. On introduit en continu 100 ml/h de sulfure de dipropyle , $10^{-4}$ mole/h de thioxanthone et $1,9.10^{-3}$ mole/h de phosphite de tributyle ainsi que, sous forme gazeuse, 120 l/h d'$H_2S$ et 60 l/h de propylène. On obtient une conversion de 74,8% de propylène et une production de 70,3 g/h de n-propylmercaptan. Un deuxième essai, identique au premier à part le débit d'$H_2S$ qui est cette fois de 300 l/h, donne une conversion de 76,9% de propylène et une production de 93,7 g/h de n-propylmercaptan.

Exemples 13 et 14

Dans le réacteur des exemples précédents, on procède à deux essais dont les paramètres suivants sont maintenus fixes: débit d'hydrogène sulfuré de 275 l/h et débit d'octène-1 de 257 g/h température de 0°C et pression de 9 bars. Les conditions particulières à chaque essaie et les résultats obtenus sont présentés dans le tableau suivant.

TABLEAU IV

| Exemple n° | Initiateurs et leurs débits en moles/h | Conversion Octène (%) | Production en octylmercaptan (g/h) |
|---|---|---|---|
| 13 | Benzophénone ($4.10^{-3}$)<br>Tributylphosphite ($4.10^{-3}$) | 53 | 158 |
| 14 | Thioxanthone ($1,4.10^{-4}$)<br>Tributylphosphite ($4.10^{-3}$) | 79,6 | 220 |

Des résultats du même ordre ont été obtenus avec des oléfines éthylène, butène-1, hexène-1 et cyclohexène.

Exemples 15 et 16

Dans le réacteur des exemples précédents, on introduit 225 $cm^3$ de méthylal, 75 $cm^3$ de maléate de dibutyle, $6.10^{-3}$ mole de benzophénone et $3{,}5.10^{-3}$ mole de tributylphosphine, ainsi que 80 litres d'hydrogène sulfuré. Ce mélange est irradié pendant 4 heures. A la fin de cette période on analyse le produit de réaction. On a effectué un deuxième essai dans les mêmes conditions, mais à la différence que la benzophénone est remplacée par de la thioxanthone ($2.10^{-4}$ mole). La réaction est conduite de façon identique.

Le tableau suivant indique les taux de conversion du maléate et les productions de mercapto-succinate de dibutyle formé par la réaction concernée:

$$BuOOC{-}CH{=}CH{-}COOBu + H_2S \longrightarrow BuOOC{-}\underset{\displaystyle SH}{\underset{|}{CH}}{-}CH_2{-}COOBu$$

TABLEAU V

| Exemple n° | Initiateurs et leurs quantités en moles | Conversion du maléate de dibutyle (%) | Mercapto-succinate de dibutyle produit (g) |
|---|---|---|---|
| 15 | Benzophénone ($6.10^{-3}$) + tributylphosphine ($3{,}5.10^{-3}$) | 90 | 51 |
| 16 | Thioxanthone ($2.10^{-4}$) + tributylphosphine ($3{,}5.10^{-3}$) | 98 | 64,5 |

Exemples 17—21

Dans l'appareil et selon le mode opératoire des exemples 1 à 5, on prépare du dodécylmercaptan avec, comme initiateurs, la dibenzo-subérone (X étant $—CH_2CH_2—$ et Y oxygène) et la thio-dibenzosubérone (même X, atome S en Y).

Les résultats suivants sont obtenues.

| Exemple n° | Initiateurs et leurs débits en moles/heure | Conversion % de dodécène | Production de dodécyl-mercaptan g/h |
|---|---|---|---|
| 17 | Dibenzosubérone $1{,}7 \times 10^{-4}$ + phosphite de tributyle $5 \times 10^{-3}$) | 78% | 258 |
| 18 | Dibenzosubérone $1{,}7 \times 10^{-4}$ + phosphite de tridécyle $6 \times 10^{-3}$ | 79% | 260 |
| 19 | Dibenzosubérone $1{,}7 \times 10^{-4}$ + tributylphosphine $3{,}5 \times 10^{-3}$ | 88% | 274 |
| 20 | Thiodibenzosubérone $1{,}7 \times 10^{-4}$ + tributylphosphine $3{,}5 \times 10^{-3}$ | 87% | 272 |
| 21 | α-éthoxy α-phényl acétophénone $2 \times 10^{-3}$ (initiateurs selon brevet U.S. 4 140 604) | 65% | 222 |

On voit que les dibenzosubérones, tout comme les dérivés xanthéniques des exemples précédents, employées à des doses beaucoup plus faibles que les initiateurs de la technique connue, donnent des résultats meilleurs que ces derniers.

Exemples 22—28

Le réacteur photochimique en pyrex est du type classique à lampe plongeante, coaxiale; son entrée en

verre est frittée à la base pour assurer une bonne diffusion des gaz hydrogène sulfuré et propylène. Le volume irradié est de 90 ml. Ce réacteur, thermostaté par une double enveloppe extérieure, fonctionne en discontinu à pression ambiante, les débits gazeux étant maintenus constants ou cours de l'expérience. Ils sont notamment de 30 l/heure pour $H_2S$ et de 30 l/h pour le propylène. Le solvant est l'éther diméthylique du diéthylène-glycol et la température à laquelle on le sature d'hydrogène sulfuré et de propylène, est de 10°C. La concentration en photosensibilisant répond à l'optimum de l'intensité absorbée dans le réacteur. La source lumineuse est une lampe à basse pression de mercure à réémission centrée sur 350 nm. Sa puissance est de 8 w. Après 30 minutes, les quantités de mercaptan formé et les % de propylène transformé ont été celles du tableau ci-après.

| Exemple n° | Initiateur et sa concentration en moles/litre | Production de propyl-mercaptan g | % de propylène converti |
|---|---|---|---|
| 22 | Dibenzosubérone 0,03 + phosphite de tributyle 0,06 | 4,38 | 20,8% |
| 23 | Dibenzosubérone 0,03 + phosphite de tridécyle 0,06 | 4,35 | 21,1% |
| 24 | Dibenzosubérone 0,03 + tributylphosphine 0,04 | 5,20 | 23 % |
| 25 | Thiodibenzosubérone 0,035 + phosphite de trioctyle 0,06 | 4,46 | 21.3% |
| 26 | Thiodibenzosubérone 0,035 + tributylphosphine 0,04 | 5,31 | 22,8% |
| 27 | α-éthoxy α-phényl acétophénone 0,09 | 3,32 | 19 % |
| 28 | Phosphite de tributyl 0,09 seul | 1,65 | 8,6% |

Ces résultate montrent que les initiateurs suivant l'invention présentent des avantages, même si la réaction est effectuée sous la pression atmosphérique, sans excès de $H_2S$ par rapport à l'oléfine.

**Revendications**

1. Procédé de synthèse de mercaptans par la réaction photochimique d'un composé oléfinique avec de l'hydrogène sulfuré, en présence d'un initiateur constitué par un composé formé de deux cycles benzéniques reliés par un pont de

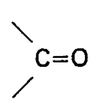 ou 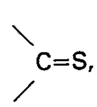,

caractérisé en ce que ce composé présente la structure de type xanthénique:

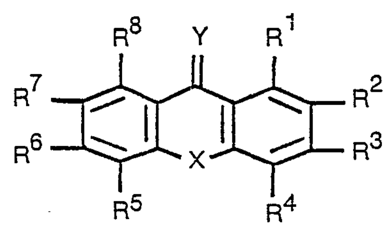

où Y est O ou S, tandis que X désigne un élément O, S ou Se, un groupe —$CH_2$—, —$CH_2CH_2$—, $SO_2$, CO, CS ou NR', R' étant un radical hydrocarboné, ou bien un groupement au phosphore tri- ou pentavalent, notamment

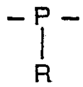, 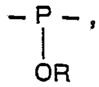, 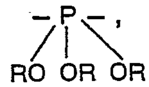, 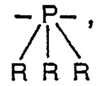,

où R désigne un alkyle, un aryle, un H ou un halogène, les symboles $R^1$ à $R^8$ désignant des atomes ou groupes semblables ou différents: H, halogène, alcoxy, alkyle ou/et aryle.

2. Procédé suivant la revendication 1, caractérisé en ce que la lumière utilisée a une longueur d'onde dans la marge de 300 à 400 nm.

3. Procédé suivant la revendication 1 ou 2, caractérisé en ce qu'un composé organique d'un élément du groupe V A de la Classification des Eléments est ajouté au milieu réactionnel conjointement avec l'initiateur du type xanthénique.

4. Procédé suivant une des revendications 1 à 3, caractérisé en ce que le milieu réactionnel contient, par litre, 0,0001 à 0,1 mole de chacun des intiateurs dans le milieu réactionnel.

5. Procédé suivant une des revendications précédentes, caractérisé en ce que la ou les composés oléfiniques traités contiennent des substituants halogène, alcool, carboxyle ou alkoxy.

6. Procédé suivant une des revendications 1 à 4, caractérisé en ce que le composé oléfinique traité est un acide ou ester non saturé, en particulier un maléate ou un fumarate d'alkyle ou/et d'aryle.

7. Procédé suivant une des revendications 3 à 6, caractérisé en ce que le composé organique des éléments du groupe V A est un phosphite ou une phosphine d'alkyle ou d'aryle

8. Procédé suivant une des revendications 3 à 6, caractérisé en ce que l'élément du groupe V A est le bismuth, l'arsenic ou l'antimoine.

9. Procédé suivant une des revendications précédentes, dans lequel le milieu réactionnel renferme un solvant, caractérisé en ce que celui-ci est un sulfure organique.

10. Procédé suivant la revendication 9, caractérisé en ce que le sulfure organique, employé en tant que solvant, est le sulfure qui se forme accessoirement dans la réaction elle-même.

11. Procédé suivant une des revendications précédentes, caractérisé en ce que l'initiateur utilisé est constitué par un ou plusieurs des composés: xanthone, xanthione, thioxanthone, dibenzosubérone ou/et thio-dibenzosubérone.

**Patentansprüche**

1. Verfahren zur Synthese von Mercaptan durch photochemische Umsetzung einer olefinischen Verbindung mit Schwefelwasserstoff in Gegenwart einer Verbindung mit zwei Benzolringen, die durch eine Brücke der Formeln

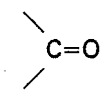 oder 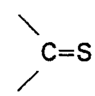

verbunden sind, als Initiator, dadurch gekennzeichnet, dass diese Verbindung eine Struktur vom Xanthentyp aufweist:

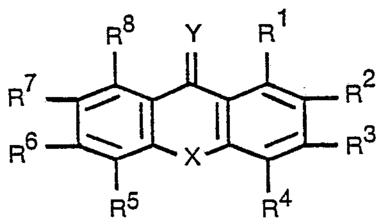

in der Y O oder S ist, während X eines der Elemente O, S oder Se oder eine der Gruppen —$CH_2$—,

—$CH_2CH_2$—, $SO_2$, CO, CS oder NR′ bedeutet, wobei R′ einen Kohlenwasserstoffrest oder eine Gruppe mit drei- oder fünfwertigem Phosphor bedeutet, insbesondere

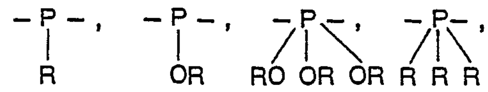

wobei R Alkyl, Aryl, H oder Halogen bedeutet, und die Symbole $R^1$ bis $R^8$ gleiche oder verschiedene Atome oder Reste bedeuten: H, Halogen, Alkoxy, Alkyl und/oder Aryl.

2. Verfahren nach Anspruch 1, dadurch gekennzeichnet, dass man Licht der Wellenlänge im Bereich von 300 bis 400 nm werwendet.

3. Verfahren nach Anspruch 1 oder 2, dadurch gekennzeichnet, dass man eine organische Verbindung eines Elements der Gruppe V A des Periodensystems dem Reaktionsmedium gleichzeitig mit dem Reaktionsinitiator vom Xanthentyp zusetzt.

4. Verfahren nach einem der Ansprüche 1 bis 3, dadurch gekennzeichnet, dass das Reaktionsmedium pro Liter 0,0001 bis 0,1 Mol eines jeden Initiators enthält.

5. Verfahren nach einem der vorstehenden Ansprüche, dadurch gekennzeichnet, dass die behandelte olefinische Verbindung (bzw. Verbindungen) Substituenten aus der Gruppe Halogen, Alkohol, Carboxyl oder Alkoxy enthält.

6. Verfahren nach einem der Ansprüche 1 bis 4, dadurch gekennzeichnet, dass es sich bei der behandelten olefinischen Verbindung um eine ungesättigte Säure oder um einen Ester davon, insbesondere um einen Maleinsäure- oder Fumarsäurealkylester und/oder -arylester, handelt.

7. Verfahren nach einem der Ansprüche 3 bis 6, dadurch gekennzeichnet, dass es sich bei der organischen Verbindung der Elemente der Gruppe V A um ein Alkyl- oder Arylphosphit oder -phosphin handelt.

8. Verfahren nach einem der Ansprüche 3 bis 6, dadurch gekennzeichnet, dass es sich bei dem Element der Gruppe V A um Wismuth, Arsen oder Antimon handelt.

9. Verfahren nach einem der vorstehenden Ansprüche, bei dem das Reaktionsmedium Lösungsmittel enthält, dadurch gekennzeichnet, dass es sich bei dem Lösungsmittel um ein organisches Sulfid handelt.

10. Verfahren nach Anspruch 9, dadurch gekennzeichnet, dass es sich dem als Lösungsmittel verwendeten organischen Sulfid um die Verbindung handelt, die als Nebenprodukt bei der Reaktion selbst gebildet wird.

11. Verfahren nach einem der vorstehenden Ansprüche, dadurch gekennzeichnet, dass man als Initiator eine oder mehrere der folgenden Verbindungen verwendet: Xanthon, Xanthion, Thioxanthon, Dibenzosuberon und/oder Thiodibenzosuberon.

## Claims

1. A process of synthesis of mercaptans by the photochemical reaction of an olefinic compound with hydrogen sulphide, in the presence of an initiator comprising a compound having two benzene rings connected by a

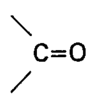 or 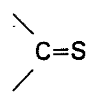

bridge, characterised in that the compound has the structure of the xanthene type:

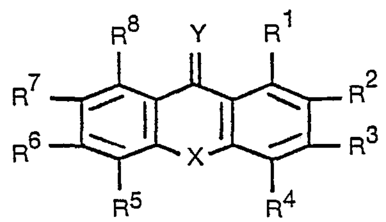

where Y is O or S, while X designates an O, S or Se element, a $—CH_2—$, $—CH_2CH_2—$, $SO_2$, CO, CS or NR′ group, R′ being a hydrocarbon group, or a tri- or pentavalent phosphorus-containing group, particularly:

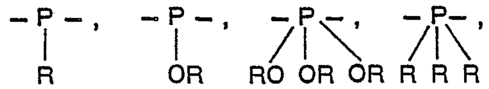

wherein R designates an alkyl, an aryl, an H or a halogen, the symbols $R^1$ to $R^8$ designating the same or different atoms or groups: H, halogen, alkoxy, alkyl and/or aryl.

2. A process according to claim 1, characterised in that the light utilized has a wavelength in the range from 300 to 400 nm.

3. A process according to claim 1 or 2, characterised in that an organic compound of an element of Group VA of the Classification of the Elements is added to the reaction medium in conjunction with the initiator of the xanthene type.

4. A process according to any of claims 1 to 3, characterised in that the reaction medium contains, per litre, 0.0001 to 0.1 mole of each of the initiators in the reaction medium.

5. A process according to any of the preceding claims, characterised in that the olefinic compound or compounds treated contain(s) halogen, alcohol, carboxyl or alkoxy substituents.

6. A process according to any of claims 1 to 4, characterised in that the olefinic compound treated is a non-saturated acid or ester, in particular an alkyl and/or aryl maleate or fumarate.

7. A process according to any of claims 3 to 6, characterised in that the organic compound of the elements of Group VA is an alkyl or aryl phosphite or phosphine.

8. A process according to any of claims 3 to 6, characterised in that the element of Group VA is bismuth, arsenic or antimony.

9. A process according to any of the preceding claims, in which the reaction medium contains a solvent, characterised in that this is an organic sulphide.

10. A process according to claim 9, characterised in that the organic sulphide employed as the solvent is the sulphide which is formed in the reaction itself.

11. A process according to any of the preceding claims, characterised in that the initiator utilized comprises one or more of the compounds xanthone, xanthione, thioxanthone, dibenzosuberone and/or thiodibenzosuberone.